Europäisches Patentamt

**European Patent Office**

**Office européen des brevets**

(19)

(11) Numéro de publication: **0 039 753**

**B1**

(12) **FASCICULE DE BREVET EUROPEEN**

(45) Date de publication du fascicule du brevet: **25.05.83**

(51) Int. Cl.³: **C 12 M 1/04**

(21) Numéro de dépôt: **80400561.9**

(22) Date de dépôt: **25.04.80**

(54) **Générateur de gaz pour cultures anaérobies.**

(43) Date de publication de la demande:
**18.11.81 Bulletin 81/46**

(45) Mention de la délivrance du brevet:
**25.05.83 Bulletin 83/21**

(84) Etats contractants désignés:
**BE CH DE GB LI LU NL**

(56) Documents cités:
**DE - A - 2 800 437**
**US - A - 4 013 422**

(73) Titulaire: **BIO MERIEUX Société anonyme dite:
Marcy l'Etoile
F-69260 Charbonnieres les Bains (FR)**

(72) Inventeur: **Trouyez, Gérard
Biomerieux Chemin de l'Orme Marcy l'Etoile
F-69260 Charbonnieres-les-Bains (FR)**

(74) Mandataire: **Nony, Michel
Cabinet Nony 29, rue Cambacérès
F-75008 Paris (FR)**

Courier Press, Leamington Spa, England.

Générateur de gaz pour cultures anaérobies

La présente invention a pour objet un nouveau générateur de gaz pour la culture de micro-organismes dans des conditions anaérobies.

Plus particulièrement, l'invention se rapporte à un générateur d'hydrogène et de gaz carbonique utilisable dans des bocaux de cultures anaérobies, tel que celui décrit par exemple dans le brevet français n° 1.525.869.

On a jégà proposé plusieurs types de générateurs de gaz, mais ceux-ci présentent divers inconvénients soit que leur réalisation présente certaines difficultés, soit que ceux-ci manquent de fiabilité.

En effet, il importe que les générateurs de gaz puissent conduire à des opérations reproductibles, ce qui n'est pas toujours le cas avec les générateurs utilisés jusqu'à ce jour.

Parmi ces générateurs qui ne donnent pas totalement satisfaction pour les raisons invoquées ci-dessus, on peut plus particulièrement citer le système connu sous la dénomination "Gaspak" commercialisé par la société Becton, Dickinson and Company consistant en une enveloppe en feuille d'aluminium contenant dans une première chambre une pastille à base de bicarbonate de sodium et une pastille de borohydrure de sodium, ces pastilles étant disposées sur un papier buvard ou filtre, ce dernier étant relié à une deuxième chambre contenant une quantité prédéterminée d'eau. Au contact de l'eau, le papier est imprégné et l'eau vient attaquer progressivement les pastilles.

On peut également mentionner en tant qu'autre dispositif générateur de gaz celui constitué d'une enveloppe en une feuille métallique contenant à sa partie inférieure un réceptacle comportant des alvéoles pour des pastilles de bicarbonate de sodium et de borohydrure de sodium, ledit réceptacle étant recouvert d'une membrane susceptible de laisser passer l'eau.

Dans la mise en oeuvre de ce dispositif, on introduit une quantité prédéterminée d'eau permettant d'immerger le réceptacle.

On a constaté que dans ce dernier dispositif le dégagement de gaz était beaucoup trop rapide compte-tenu de la grande perméabilité de la membrane et que de plus, au moment du conditionnement il existait des risques non négligeables de perforations de la membrane.

Compte-tenu de ces différents inconvénients des générateurs connus, la Société déposante vient de mettre au point un nouveau dispositif permettant de remédier aux inconvénients des dispositifs utilisés jusqu'à ce jour.

Le dispositif selon la présente invention permet d'une part d'éviter un dégagement trop rapide d'hydrogène et d'autre part d'assurer un dégagement total du gaz carbonique.

En effet, on a constaté qu'il était tout à fait souhaitable que le dégagement d'hydrogène s'effectue au début plus lentement que le dégagement de gaz carbonique.

La présente invention a pour objet, à titre de dispositif nouveau, un générateur de gaz pour cultures anaérobies, comprenant une enveloppe hermétique, ouvrable à sa partie supérieure pour l'introduction d'eau et le dégagement subséquent du gaz, ladite enveloppe comportant à sa partie inférieure en position verticale une boîte rigide non étanche à l'eau, contenant à sa base au moins une pastille génératrice de gaz carbonique et, supportée par ladite pastille, une pastille génératrice d'hydrogène.

Lors de l'utilisation du générateur de gaz selon l'invention on introduit dans l'enveloppe une quantité prédédeterminée d'eau de telle sorte que celle-ci pénètre par capillarité dans la boîte rigide de façon à imprégner la pastille génératrice de $CO_2$ sans toutefois imprégner la pastille génératice d'hydrogène.

De façon préférentielle, la quantité d'eau introduite est telle que celle-ci n'imprègne que la moitié aus plus de la pastille génératrice de gaz carbonique.

Dès l'introduction de l'eau dans le générateur de gaz, la pastille génératrice de $CO_2$ commence à se désagréger à sa partie inférieure ce qui entraîne progressivement son affaissement, entraînant dans sa chute la pastille génératrice d'hydrogène qui vient ainsi qu contact de l'eau, ce qui entraîne alors le dégagement subséquent de l'hydrogène.

Dès le début du dégagement du $CO_2$ il se produit à l'intérieur de la boîte rigide une légère surpression ce qui permet d'abaisser le niveau de l'eau par rapport au niveau extérieur et ainsi de limiter si nécessaire l'attaque de la pastille génératrice d'hydrogène.

Le générateur de gaz selon l'invention présente par ailleurs du fait de sa structure l'avantage d'éviter que les pastilles, lors du stockage ou de la manipulation du générateur, ne viennent à se broyer.

En effet, comme indiqué ci-dessus, les pastilles sont conditionnées dans une boîte rigide, de telle sorte qu'en cas de choc celles-ci ne peuvent être endommagées.

De façon à maintenir la boîte rigide dans le fond de l'enveloppe, en position de fonctionnement, il est souhaitable que l'enveloppe comporte, à la partie supérieure de la boîte rigide un moyen de maintien par exemple sous forme d'un rétrécissement réalisé, soit par soudure, soit par collage des deux parties formant l'enveloppe.

Selon une forme préférée de l'invention, la boîte rigide contient deux pastilles génératrices de gaz carbonique disposées de façon symétrique à la base inférieure de ladite boîte, la pastille génératrice d'hydrogène étant supportée par les deux pastilles génératrices de gaz carbonique.

Par ailleurs, selon une autre forme préférée de l'invention, les pastilles peuvent être maintenues en position par des moyens tels que plots, nervures ou reliefs, soit solidaires du fond de la boîte, soit solidaires du couvercle, ceci de façon à éviter que les pastilles de changent de position dans la boîte rigide ou ne viennent à se détériorer par contact entre elles.

Afin d'interdire toute fermeture étanche de la boîte rigide contenant les pastilles, celle-ci peut comporter divers moyens permettant de favoriser le passage de l'eau, par exemple un ou plusieurs épaulements ou ouvertures, réalisés à la base de la boîte, ou encore un ou plusieurs épaulements réalisés sur le bord intérieur du couvercle ou sur les flancs extérieurs du corps de la boîte.

Selon l'invention, la boîte rigide peut prendre diverses formes notamment une forme rectangulaire et comporter, comme indiqué ci-dessus, des moyens de maintien pour les pastilles, ces moyens pouvant être en un nombre variable, par exemple de quatre ou cinq.

La boîte rigide contenant les pastilles peut être réalisée en matière plastique transparente, présentant une bonne rigidité et étant facilement moulable, à cet égard on peut en particulier citer comme matière plastique particulièrement appropriée, le polystyrène cristal, le polyméthacrylate de méthyle, etc.

Selon l'invention, l'enveloppe peut être réalisée en matière souple, notamment en matière plastique transparente telle qu'en une feuille de polyéthylène, ce qui permet, par simple visualisation, de vérifier le bon niveau de l'eau dans la boîte rigide.

Toutefois, l'enveloppe peut également être réalisée en une feuille métallique, en particulier en une feuille d'aluminium.

Bien que diverses substances puissent être employées pour produire du gaz carbonique et de l'hydrogène, on utilise de préférence selon l'invention d'une part du bicarbonate de sodium éventuellement en mélange avec un acide tel que l'acide tartrique on l'acide citrique et d'autre part du borohydrure de sodium ou de potassium.

D'autres caractéristiques et avantages ressortiront de la description qui va suivre du générateur de gaz selon l'invention, description donnée à titre d'exemple et en regard du dessin annexé sur lequel:

— la figure 1 représente une vue en élévation avec coupe partielle du générateur selon l'invention,
— la figure 2 représente une vue en coupe selon A—A' de la figure 1,
— la figure 3 représente une vue éclatée en perspective de 3/4 de la boîte rigide contenant les pastilles.

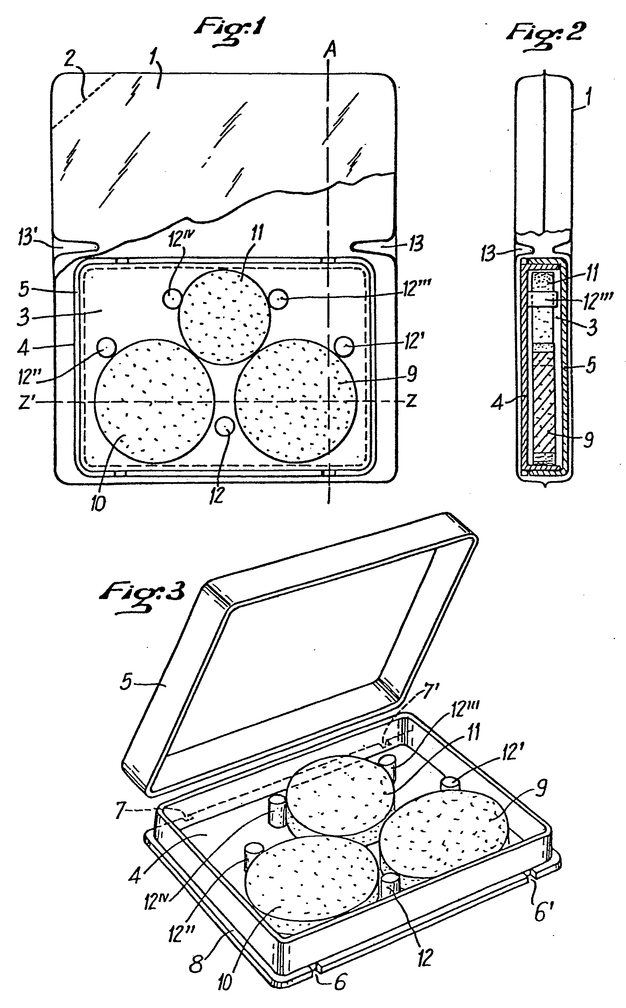

Le générateur représenté à la figure 1 comporte une enveloppe (1) réalisée en une matière transparente ou en une feuille métallique, dont l'ouverture est réalisée par coupure selon les pointillés indiqués en (2).

A la partie inférieure de l'enveloppe on a représenté une boîte rigide (3) qui peut être en matière plastique transparente, cette boîte étant constituée d'un corps (4) et d'un couvercle (5) ledit couvercle s'adaptant sur le corps de façon non-étanche à l'eau.

On a représenté aux figures 1 et 3, des ouvertures (6—6'), (7—7'), réalisées sur le bord périphérique (8) du corps de la boîte, ce qui permet d'assurer la non-étanchéité de la boîte et par ailleurs un bon dégagement du gaz.

On a également représenté dans la boîte (3), deux pastilles (9), et (10), de $CO_3H$ Na et une pastille de borohydrure de Na (11), cette dernière étant supportée dans la boîte par les deux pastilles de bicarbonate de Na.

Des plots (12), (12'), (12''), etc., solidaires du fond de la boîte (3) permettant de maintenir en position les pastilles (9), (10), et (11).

Les figures 1 et 2 montrent également les rétrécissements (13) et (13') permettant de bien positionner la boîter rigide (3) dans le fond de l'enveloppe (1).

Les figures 1 à 3 décrites ci-dessus ne représentent qu'une forme particulière de réalisation de l'invention, et il est bien entendu tout à fait possible d'utiliser par exemple d'autres formes de boîte rigide, notamment des boîtes de forme carrée ou triangulaire.

Dans la mise en ouvre du dispositif générateur de gaz selon les figures 1 à 3, on pratique tout d'abord une ouverture à la partie supérieure de l'enveloppe selon les pointillés représentés en (2) de la figure 1.

On introduit ensuite une quantité prédéterminée de'eau, tel que représenté par la ligne horizontale Z—Z' de la figure 1.

L'eau pénètre alors dans la boîte rigide (3) par capillarité et vient imprégner les pastilles de bicarbonate de sodium (9) et (10). Il se produit instantanément un dégagement de gaz carbonique qui s'échappe par le haut et les côtés de la boîte rigide (3).

Au fur et à mesure de la décomposition des pastilles (9) et (10) de bicarbonate de sodium, la pastille (1) de borohydrure de sodium descend et vient ensuite au contact de l'eau (Z—Z') ce qui a pour effet d'engendrer le dégagement subséquent d'hydrogène.

Pour cela il importe bien entendu que le plot de maintien (12) représenté aux figures 1 et 3 soit placé le plus bas possible de façon à faciliter la descente de la pastille (11).

Il est cependant nécessaire que ce plot (12) soit positionné dans la boîte rigide de telle sorte qu'il ne puisse se produire de contact entre les deux pastilles de bicarbonate de sodium (9) et (10), ceci de façon à éviter que celles-ci ne se détériorent.

## Revendications

1. Générateur de gaz pour cultures anaérobies, comprenant une enveloppe hermétique, ouvrable à sa partie supérieure pour l'introduction d'eau et le dégagement subséquent du gaz, ladite enveloppe contenant des pastilles génératrices de gaz, caractérisé par le fait que l'enveloppe comporte à sa partie inférieure, en position verticale, une boîte rigide non-étanche à l'eau, contenant à sa base au moins une pastille génératrice de gaz carbonique, et, supportée par ladite pastille, une pastille génératrice d'hydrogène.

2. Générateur selon la revendication 1, caractérisé par le fait que l'enveloppe comporte à la partie supérieure de la boîte rigide un moyen pour la maintenir au fond de ladite enveloppe.

3. Générateur selon l'une quelconque des revendications précédentes, caractérisé par le fait que la boîte rigide contient deux pastilles génératrices de gaz carbonique disposées de façon symétrique à la base inférieure de ladite boîte, la pastille génératrice d'hydrogène étant supportée par lesdites deux pastilles.

4. Générateur selon l'une quelconque des revendications précédentes, caractérisé par le fait que la boîte rigide possède des moyens pour le maintien en position des pastilles.

5. Générateur selon la revendication 4, caractérisé par le fait que les moyens de maintien des pastilles sont solidaires du fond de la boîte.

6. Générateur selon l'une quelconque des revendications précédentes, caractérise par le fait que la boîte rigide comporte au moins un moyen permettant de favoriser le passage de l'eau.

7. Générateur selon l'une quelconque des revendications précédentes, caractérise par le fait que la boîte rigide a une forme rectangulaire.

8. Générateur selon l'une quelconque des revendications précédentes, caractérisé par le fait que l'enveloppe est réalisée en matière souple.

9. Générateur selon l'une quelconque des revendications précédentes, caractérisé par le fait que l'enveloppe est en matière plastique transparente.

10. Générateur selon la revendication 1, caractérisé par le fait que l'enveloppe est en une feuille métallique par exemple en une feuille d'aluminium.

11. Générateur selon l'une quelconque des revendications précédentes, caractérisé par le fait que la pastille génératrice de gaz carbonique est constituée de bicarbonate de sodium, éventuellement en mélange avec un acide tel que l'acide tartrique ou l'acide citrique.

12. Générateur selon l'une quelconque des revendications 1 à 10, caractérise par le fait que la pastille génératrice d'hydrogène est constituée de borohydrure de sodium ou de potassium.

## Patentansprüche

1. Gaserzeuger für anaerobe Kulturen mit einem luftdichten Gehäuse, das an seinem Oberteil geöffnet werden kann um Wasser hinein- und danach Gas herauszulassen, wobei das Gehäuse die gaserzeugenden Pastillen erhält, dadurch gekennzeichnet, daß des Gehäuse in seinem Unterteil vertikal angeordnet einen steifen, wasserdurchlässigen Behälter aufweist, der an seinem Boden mindestens eine kohlendioxiderzeugende Pastille enthält auf der eine wasserstofferzeugende Pastille liegt.

2. Gaserzeuger nach Anspruch 1, dadurch gekennzeichnet, daß das Gehäuse in einer Region oberhalb des steifen Behälters Mittel aufweist, um diesen am Boden des Gehäuses zu halten.

3. Gaserzeuger nach einem der vorhergehenden Ansprüche, dadurch gekennzeichnet, daß der steife Behälter zwei kohlendioxiderzeugende Pastillen enthält, die in symmetrischer Anordnung am Boden im unteren Teil des Behälters angeordnet sind, wobei die wasserstofferzeugende Pastille durch die beiden anderen Pastillen gehalten wird.

4. Gaserzeuger nach einem der vorhergehenden Ansprüche, dadurch gekennzeichnet, daß der steife Behälter Mittel zum In-Position-Halten der Pastillen aufweist.

5. Gaserzeuger nach Anspruch 4, dadurch gekennzeichnet, daß die Mittel zum Halten der Pastillen mit dem Boden des Behälters verbunden sind.

6. Gaserzeuger nach einem der vorhergehenden Ansprüche, dadurch gekennzeichnet, daß der steife Behälter mindestens eine Vorrichtung aufweist, die das Durchströmen von Wasser ermöglicht.

7. Gaserzeuger nach einem der vorhergehenden Ansprüche, dadurch gekennzeichnet, daß der steife Behälter eine rechteckige Form aufweist.

8. Gaserzeuger nach einem der vorhergehenden Ansprüche, dadurch gekennzeichnet, daß das Gehäuse aus einem biegsamen Stoff gefertigt ist.

9. Gaserzeuger nach einem der vorhergehenden Ansprüche, dadurch gekennzeichnet, daß das Gehäuse aus transparentem Kunststoffmaterial gefertigt ist.

10. Gaserzeuger nach Anspruch 1, dadurch gekennzeichnet, daß das Gehäuse aus Metallblech, beispielsweise aus Aluminiumblech besteht.

11. Gaserzeuger nach einem der vorhergehenden Ansprüche, dadurch gekennzeichnet, daß die kohlendioxiderzeugende Pastille aus Natriumbicarbonat, gegebenenfalls in Mischung mit einer Säure wie Weinsäure oder Zitronensäure, besteht.

12. Gaserzeuger nach einem der Ansprüche 1—10, dadurch gekennzeichnet, daß die wasserstofferzeugende Pastille aus Natrium- oder Kaliumborhydrid besteht.

## Claims

1. A gas generator for anaerobic cultures, comprising an airtight envelope which can be opened at the upper part of it for the introduction of water and the subsequent release of the gas, the said envelope containing gas-generating pastilles, characterized by the fact that the envelope includes in the lower portion of it and in a vertical position a rigid non-watertight box which contains at the foot of it at least one pastille generative of carbon dioxide gas and supported by the said pastille, a pastille generative of hydrogen.

2. A generator as in Claim 1, characterized by the fact that the envelope includes at the upper portion of the rigid box a means of keeping it at the bottom of the said envelope.

3. A generator as in either of the preceding claims, characterized by the fact that the rigid box contains two pastilles generative of carbon dioxide gas, arranged symmetrically at the foot of the said box, the pastille generative of hydrogen being supported by the said two pastilles.

4. A generator as in any one of the preceding claims, characterized by the fact that the rigid box has means of retention of the pastilles in position.

5. A generator as in Claim 4, characterized by the fact that the means of retention of the pastilles are integral with the bottom of the box.

6. A generator as in any one of the preceding claims, characterized by the fact that the rigid box includes at least one means enabling the flow of the water to be favoured.

7. A generator as in any one of the preceding claims, characterized by the fact that the rigid box has a rectangular shape.

8. A generator as in any one of the preceding claims, characterized by the fact that the envelope is produced from flexible material.

9. A generator as in any one of the preceding claims, characterized by the fact that the evenlope is of transparent plastic matter.

10. A generator as in Claim 1, characterized by the fact that the envelope is of metal foil, for example, of aluminium foil.

11. A generator as in any one of the preceding claims, characterized by the fact that the pastille generative of carbon dioxide gas consists of sodium bicarbonate, possibly in a mixture with an acid such as tartaric acid or citric acid.

12. A generator as in any one of the Claims 1 to 10, characterized by the fact that the pastille generative of hydrogen consists of sodium or potassium borohydride.

Fig.1

Fig.2

Fig.3